# EUROPEAN PATENT SPECIFICATION

(11) **EP 0 605 509 B1**
(45) Date of publication and mention of the grant of the patent: **20.12.1995**
(21) Application number: 92919648.3
(22) Date of filing: 16.09.1992
(51) Int. Cl.: C09K 15/34, A23L 1/221, A23L 3/3463

(54) **ANTIOXIDANT OLEORESIN COMPOSITIONS AND A PROCESS FOR THEIR PRODUCTION**
ANTIOXIDAN-OLEORENINZUSAMMENSETZUNG UND VERFAHREN ZUR HERSTELLUNG
COMPOSITIONS OLEORESINEUSES ANTIOXYDANTES ET PROCEDE DE PREPARATION

(30) Priority: 20.09.1991 US 763158
(43) Date of publication of application: 13.07.1994
(73) Proprietor: NORAC TECHNOLOGIES INC., Edmonton, Alberta T6E 5V2 (CA)
(72) Inventor: NGUYEN, Uy, Edmonton, Alberta T6L 3V2 (CA); FRAKMAN, Grigory, Edmonton, Alberta T6M 1X1 (CA); EVANS, David A., Edmonton, Alberta T6J 4V6 (CA); MOSER, Daniel G. c/o Norac Technologies Inc., Edmonton, Alberta T6J 4V6 (CA)
(74) Representative: Fürniss, Peter, Dr.
(86) International application number: CA9200399
(87) International publication number: WO9306190

(56) References cited:
- EP-A- 0 038 959
- EP-A- 0 480 077
- DE-A- 4 026 438
- US-A- 3 950 266
- US-A- 4 450 097
- US-A- 5 017 397
- DATABASE WPIL, Derwent Publications Ltd., London, GB; AN 87-074946; & JP-A-62 026 293

## Description

This invention relates to the production of natural antioxidant extracts with improved antioxidant activity by concentrating the content of carnosic acid in such extracts.

Antioxidants are commonly used in the food, beverage and cosmetic industries to prevent or retard deterioration of flavor, aroma or color resulting from oxidative processes. Recently there has been concern that many antioxidants used today are synthetic chemicals, and this concern has been associated with industry and consumer demand for "all natural" ingredients particularly in food and cosmetic products.

This demand for "all natural" ingredients has focused attention on certain natural antioxidants from plant sources. The most important of these has been natural extracts of the Labiatae spices, rosemary and sage. Unfortunately, methods of concentrating the antioxidant principal, carnosic acid, from these spices as revealed by the prior art, have not been successful in producing natural antioxidant extracts which can compete with the synthetic products in antioxidant activity.

More recently, there has been increased interest from the health-care and medical industries in the role of natural antioxidants as inhibitors of in vivo lipid oxidation through their activities as oxygen or free radical scavengers. Such properties are thought to play a role in the body's natural defences against both cancer and heart disease.

There is a clearly defined need for a method to selectively concentrate the carnosic acid content and other natural phenolic diterpene antioxidant compounds of natural herbal materials and their extracts. Such preferred extracts should show enhanced antioxidant activity as well as the absence of green coloration and undesirable flavors and aromas, and be soluble in oils, fats and ethyl alcohol to allow easy incorporation into end products.

### STATE OF THE ART

Chipault et al. (Food Research 17:46, 1952, and Food Technology 10(5):209, 1956) showed that many ground spices and their organic solvent extracts exhibited antioxidant activity with the Labiatae members rosemary and sage being strongest.

Brieskorn et al. (J. Org. Chem. 29:2293, 1964) isolated a phenolic diterpenic lactone of the ferruginol type from leaves of Rosmarinus officinalis and Salvia officinalis which was shown to be carnosol. Later however, Wenkert et al. (J. Org. Chem. 30:2931, 1965) showed that the major terpenic constituent of rosemary leaves was carnosic acid and that carnosol was an artificially produced derivative resulting from the ready oxidative conversion from carnosic acid. In 1969 Brieskorn and Domling (Zeitschrift fuer Lebensmitteluntersuchung und-forschung 141(1):10, 1969) showed that carnosic acid and carnosol were excellent antioxidants and that the antioxidant property of rosemary and sage was caused by the presence of carnosic acid in the leaves of these plants. The antioxidant activity of carnosic acid and carnosol was shown to be equivalent to the synthetic antioxidant BHT.

A recent review by Schuler ("Natural Antioxidants Exploited Commercially", Food Antioxidants, B.J.F. Hudson, Ed., 1990) based on unpublished results by Pongracz et.al (1978-87) confirms the antioxidant effectiveness of carnosic acid either in the form of a commercial rosemary extract (approx. 16% carnosic acid), an experimental extract (24.8% carnosic acid) or synthetic carnosic acid. Antioxidant activity as measured by the Rancimat Test using lard and peanut oil was directly related to carnosic acid content. In both the lard and the oil systems, carnosic acid was superior to both synthetic antioxidants BHA and BHT at a usage of 200 ppm.

A natural Labiatae herb extract with preferred antioxidant properties would be one containing the highest concentration of the natural antioxidant compound carnosic acid. For convenience in application, the preferred extract should be oil and fat soluble and in some cases soluble in food-grade grain alcohol. It is most important that the preferred extract be low in essential oil components which could impart unwanted flavor and aroma characteristics from the herb to the end product.

A review of the prior art reveals that existing Labiatae herb extracts with antioxidant properties suffer from a number of deficiencies as additives for commercial products. These include: the non-selective extraction of the Labiatae herbs resulting in a low concentration of antioxidant compounds in the extract, the presence of unwanted essential oil components which must be removed by an additional processing step, the formation of the oxidative derivative carnosol from carnosic acid as a consequence of chemical methods of preparation, and the further dilution of antioxidant concentration in the extract resulting from the addition of mono-, di- and triglycerides during extraction to enhance solubility or to act as a carrier for distillation or molecular distillation. This latter dilution further inhibits solubility in food-grade and cosmetic-grade grain ethanol.

Chang (U.S. Pat. No. 3,950,266) discloses the use of organic solvents such as ethyl ether and methanol to extract rosemary and sage leaves. The extract then requires further dilution in vegetable oil prior to vacuum or molecular distillation to remove essential oil flavor and aroma components. Wu et al. (JAOCS 59(8):339 1982), following a modified method of Chang, demonstrated that the major antioxidant compound in the extract was carnosol which was tasteless as compared with the known bitterness of rosemary and sage antioxidant extracts. Clearly, the natural carnosic acid had been oxidised to the derivative carnosol as a result of the process.

Kimura (U.S. Patent No. 4,380,586) discloses the use of ethanol hexane mixtures while Aesbach and Philippossian (Swiss Patent No. 672,048,A5) describes the use of hexane followed by ethanol extraction of the residue. Inatani et al. (Agric. Biol. Chem. 47(3):521, 1983) using a similar procedure (hexane/ethanol) found the major active antioxidant component to be the oxidative derivative carnosol.

Todd (U.S. Patent No. 4,877,635) discloses the use of acetone or other organic solvents followed by acetone with a precipitation step to remove acetone insoluble pro-oxidant substances. In a preferred embodiment, the extract is diluted 50% with mono-, di-, and triglycerides during processing. Steam distillation is still proposed to remove essential oil components.

Kimura (U.S. Patent No. 4,363,823) extracts fresh or previously steam distilled herb with alcohol and then evaporates the alcohol to form a paste product. Further treatment with boiling water may follow to collect the water insoluble precipitate which can then be dried. The resulting product is low in antioxidant activity due to the poorly selective nature of the alcohol solvent.

Berner (U.S. Patent No. 3,732,111) extracts the ground dry spice with hot edible oil and removes the essential oil with steam under vacuum, resulting in a diluted extract of the antioxidant compounds which cannot be concentrated. The product is not ethanol soluble.

Tateo and Fellin (Perfumer and Flavorist 13:48, 1988) describe the use of supercritical carbon dioxide to remove the essential oil from rosemary leaves after which the residue is extracted with ethanol. Effectiveness of the extract was equivalent to a commercial extract prepared using organic solvents but was less than BHA at the same level of usage (0.03%).

CAL/Pfizer (ca 1988) advertise a commercial product (Rosemary Deodorised) in which the oleoresin fraction obtained by extracting rosemary with supercritical carbon dioxide is subjected to molecular distillation to remove essential oil and improve color and flavor. The resulting product contains about 80% capric and caprylic triglyceride (the distillation carrier) and has poor oil solubility and relatively weak antioxidant activity.

More recently Nguyen et al. (U.S. Patent No. 5,017,397) disclose a process for the extraction of antioxidants from Labiatae herbs using supercritical carbon dioxide as the solvent and effecting simultaneous removal of the essential oil component. Nguyen et al. (US Patent Appln. No. 670,760) disclose that the extracted oleoresins by this preferred process contain some five-times the quantity of carnosic acid as obtained by prior art processes. However, in the case of rosemary and sage raw materials, not all the carnosic acid is extracted and the oleoresin has a green coloration.

Frakman et al. (1991 JOACS to be published) describe a laboratory HPLC analytical procedure for the measurement of carnosic acid in both herbal materials and extracts. Using this process, Nguyen et al. have shown that samples of commercial rosemary antioxidants produced by prior art processes contain only approximately 5% by weight of carnosic acid. In contrast, the supercritical process of Nguyen et al. produces antioxidant extracts from rosemary or sage containing 20-30% carnosic acid.

It is clear there is a need for a process capable of selectively extracting or concentrating the carnosic acid content of carnosic acid-containing herbal materials and their extracts.

### BRIEF SUMMARY OF THE INVENTION

The herbal starting material containing carnosic acid or an extract thereof is subjected to the following steps:
1. The starting material is mixed with a 40% - 95% by weight solution of ethanol or methanol in water and the solution is separated and recovered from the insoluble material.
2. The alcohol concentration of the solution is then adjusted to induce precipitation of the oleoresin. This precipitate, which contains the carnosic acid, is separated from the solution and recovered.

The extraction in Step 1 may be conveniently carried out in any standard mixing or percolating device and repeated any number of times. In the case of direct extraction of herbal material the herbal material is preferably ground or comminuted prior to extraction. The separation steps can be effected by filtration, centrifuging or equivalent procedures. The concentrations of alcohol in water as specified must take account of any water in the alcohol used and any water or alcohol that may be present in a herbal extract. The adjustment of the alcohol concentration in Step 2 is preferably accomplished by evaporation or distillation since it results in recovery of the alcohol which may then be re-used. The precipitated extract obtained in Step 2 may be used directly or dried.

The invention results in extraction of not less than 75% of the available carnosic acid and provides natural herbal extracts which are increased in carnosic acid content as compared to the starting material. The resulting extracts are substantially free of green coloration and herbal flavors and aromas. The extracts are soluble for the use intended in oils and fats and food-grade grain alcohol. The extracts exhibit enhanced antioxidant activity as compared to the starting materials.

### DESCRIPTION OF BEST MODE

The invention is a process for producing antioxidant oleoresins by extracting or concentrating carnosic acid from herbal materials or extracts thereof which contain carnosic acid. As carnosic acid is a known powerful natural antioxidant, such oleoresins as are produced by the invention have great economic utility in the food, beverage, cosmetic and pharmaceutical industries. The antioxidant oleoresins produced are substantially free of green coloration and herbal flavors and aromas and are soluble in fats, oils and grain alcohol and are suitable for incorporation into food, beverage, cosmetic and pharmaceutical products.

In accordance with the invention, herbal materials containing carnosic acid, such as for example the leaves of the Labiatae domestic herbs rosemary (Rosmarinus spp.) and sage (Salvia spp.) or carnosic acid-containing extracts thereof, are mixed with a solution of 40%-60% by weight of ethyl alcohol in water, or a 60%-80% solution of methanol in water, and the solution is separated from the insoluble material and recovered. This step may be repeated a number of times. The concentration of the alcohol in the extract solution is then adjusted to less than 40%, preferably less than 20%, in the case of ethanol; or less than 60%, preferably less than 40%, in the case of methanol, by the removal of alcohol or the addition of water, which induces precipitation of the oleoresin containing the carnosic acid. The oleoresin precipitate is then separated from the solution and can be dried.

According to another aspect of the invention, the first step comprising mixing the raw material with the solution of alcohol in water may be carried out in two steps by first mixing the raw material with an alcohol/water solution of greater than 60%-80% alcohol (for example, 95%) and then adjusting the alcohol concentration to the desired range by the addition of water. After mixing with the higher strength solvent an optional separation of insoluble material may be carried out prior to dilution. Again these two steps may be repeated any number of times.

In the case where the starting material comprises herbal material it is preferable to grind or comminute the material prior to extraction.

The separations of solution from insolubles and of precipitate from solution can be effected by filtration, centrifuging, decanting or equivalent procedures.

The adjustment of the alcohol concentration to less than 40% (ethanol) or less than 60% (methanol) is preferably effected by removal of the alcohol, such as for example by evaporation or distillation, which allows recovery of the alcohol.

The concentration of alcohol in water specified for extraction should take account of the water content of the herbal material and also should take account of any water and alcohol which may be present in a herbal extract. For example, herbal materials are often extracted with, for example, 95% ethyl alcohol in water. The present invention may be applied to such extract solutions by the simple addition of water to bring the alcohol concentration within the specified range of 40%-60% in water and then proceeding in accordance with the invention.

While a preferred starting material is ground dry rosemary or sage leaves, the raw material may comprise ground leaf residue from prior extraction by steam distillation, by subcritical or supercritical carbon dioxide, by organic solvents or by other extraction processes, providing only that the residue still contains some carnosic acid.

Natural extracts containing carnosic acid may themselves be starting materials for the process. Such extracts include oleoresins of rosemary or sage produced with organic solvents or carbon dioxide. By means of the present invention the carnosic acid content of these starting materials may be concentrated some 2-10 fold while any green coloration is simultaneously removed.

It has been the surprising result of this invention that carnosic acid is substantially soluble in 40%-60% (and higher) solutions of ethyl alcohol in water and in 60%-80% solutions of methyl alcohol in water but is substantially insoluble in solutions comprising less than 40% of the alcohol. This result is unexpected. These alcohol solutions in water are not sufficiently high in the alcohol content to dissolve green coloring matter that may be present in the original material, but they form very powerful solvents for carnosic acid. Indeed the solvating capacity of the specified concentrations is such that the carnosic acid in the starting material is more than 80% dissolved in 30-60 seconds of mixing at room temperature.

Extraction solutions containing in excess of 60% ethyl alcohol or 80% methyl alcohol result in the undesirable extraction of green coloring matter together with flavor and aroma compounds and other diluent materials. Extraction solutions containing less than 40% ethyl alcohol or 60% methyl alcohol are not capable of efficient carnosic acid extraction and remove only 60% or less of the original carnosic acid. These low alcohol content extraction solutions allow an oxidative deterioration of the carnosic acid. Within the concentration range of 40%-60% ethyl alcohol and 60%-80% methyl alcohol, over 80% of the carnosic acid is extracted within 60 seconds yet no green coloration and very little flavor and aroma goes into solution.

A further novel aspect of the invention is the effectiveness of the alcohol dilution in water to precipitate the oleoresin containing the carnosic acid. To recover the precipitate quantitatively dilution of the solvent to less than 20% in the case of ethanol or less than 40% in the case of methanol is preferred.

The precipitated antioxidant oleoresins produced by the invention are recovered as light brown pastes which may be dried or incorporated directly into oils, fats, foods and other end-products. When derived from, for example, rosemary or sage leaves containing approximately 3% carnosic acid, the resulting oleoresins will contain typically 30% - 40% carnosic acid on a dry matter basis. When derived from, for example, a green colored supercritical antioxidant oleoresin produced from rosemary leaves and containing approximately 25% carnosic acid, the oleoresins resulting from the process of the invention will contain typically 50%-80% carnosic acid on a dry matter basis.

A further novel aspect of the subject invention is that other natural phenolic diterpene antioxidant compounds, such as for example, methyl carnosate and carnosol, which may be present in the starting material are simultaneously increased in concentration in the extracted oleoresin produced by the method of the invention.

The present invention is clearly effective in the selective extraction and concentration of carnosic acid from herbal materials and their extracts, and allows the production of oleoresins with the highest content of natural carnosic acid known to date.

The invention will be more fully understood by reference to the following examples; however, these examples are merely intended to illustrate embodiments of the invention and are not to be construed as limiting the scope of the invention.

### EXAMPLE 1

### SELECTIVE EXTRACTION

Approximately 5 gram samples of ground rosemary leaves (75% less than 0.42 mm (40 mesh)) known to contain approximately 3% carnosic acid by the method of Frakman et al. (1991) were each subjected to stirring for two minutes at room temperature in approximately 40 grams of ethyl alcohol/water solution varying in strength from 40% to 95% by weight of the alcohol. At the end of two minutes stirring the solution was separated from the insolubles by filtration through laboratory filter paper. The resulting solutions were then evaluated subjectively for green coloration and for carnosic acid content by the method of Frakman et al. (1991). The results are shown in Table 1.

**TABLE 1**

| Ethanol/Water Concentration | Color | Carnosic Acid Recovery (%) |
|---|---|---|
| 95% | Green | 98.7% |
| 60% | Pale Green | 95.4% |
| 55% | Amber-Pale Green | 92.7% |
| 50% | Amber | 83.8% |
| 40% | Amber | 55.7% |

Table 1 demonstrates that the majority of the carnosic acid is extracted into the solution without concomitant green coloration when the ethanol/water concentration of the solution is in the range of 40%-60% ethanol and optimally is in the range of 50%-55%.

### EXAMPLE 2

### SELECTIVE PRECIPITATION

Approximately 10 gram samples of ground rosemary leaves containing approximately 3% carnosic acid by the method of Frakman et al. (1991) were each subjected to stirring for one minute at room temperature in 80 grams of 55% by weight ethanol/water solution. The mixtures were filtered through laboratory filter paper to remove insolubles. The resulting solutions were then each evaporated on a laboratory Rotavapor to reduce the ethanol concentration of the solutions stepwise to zero. After the appropriate amount of ethanol had been removed each solution was centrifuged in a laboratory centrifuge at 4000 RPM for 20 minutes to recover the precipitated oleoresin. The oleoresins were dried in a vacuum oven and analysed for carnosic acid content by the method of Frakman et al. (1991). Results are shown in Table 2.

**TABLE 2**

| Ethanol/Water Concentration | Carnosic Acid in Oleoresin |
|---|---|
| 39.72% | 9.89% |
| 30.62% | 26.22% |
| 20.87% | 35.51% |
| 11.59% | 27.16% |
| 0.00% | 36.53% |

Table 2 demonstrates that reduction of the ethanol/water concentration to less than 40% will induce precipitation of some carnosic acid while reduction to less than 20% is sufficient to maximize the yield of carnosic acid in the oleoresin. Reduction to 0% is preferable however as recovery of ethanol is maximized.

### EXAMPLE 3

### HERBAL MATERIAL

Approximately 10 grams of ground rosemary leaves (75% less than 40 mesh) containing approximately 3.3% carnosic acid by the method of Frakman et al. (1991) were mixed with a magnetic stirrer in a beaker with 80 grams of 80% by weight methyl alcohol in water for 2 minutes at 18 °C. The mixture was then passed through a glass filter under vacuum to separate the solution from the insolubles. The solution obtained was clear amber in color. The solution was then placed in a laboratory Rotavapor and evaporated until all the methanol had been removed. During evaporation a light yellow-brown precipitate formed in the solution. The resulting aqueous solution and precipitate was centrifuged in a laboratory centrifuge at 4000 RPM for 20 minutes. The aqueous fraction was decanted off the precipitated pale yellow-brown paste which was then dried in a vacuum oven to less than 10% moisture content. The dried oleoresin yielded 6.1 grams or 61% of the original leaf material. On analysis by the method of Frakman et al. (1991) the dried oleoresin was found to contain 41.1% carnosic acid. The method was effective in extracting and recovering 75% of the carnosic acid in the starting material and effecting a 12-fold increase in carnosic acid concentration.

### EXAMPLE 4

### HERBAL EXTRACT

20.05 grams of a light green rosemary extract produced by the method of Nguyen et al. and containing 25.3% carnosic acid by the method of Frakman et al. (1991) was mechanically stirred in a beaker with 156.12 grams of 95% ethanol in water for 1 hour at 50 °C. after which 156 grams of water was added to the mixture. The mixture was cooled to room temperature and filtered through a glass filter under vacuum to separate the solution from the insolubles. The solution was a light amber in color. The solution was evaporated in a laboratory Rotavapor to remove the ethanol and precipitate a light brown oleoresin. The resulting aqueous solution and precipitate was centrifuged in a laboratory centrifuge at 4000 RPM for 20 minutes and the solution decanted off the precipitated paste which was dried in a vacuum oven to less than 10% moisture. The dried light-brown oleoresin was found to contain 60.1% carnosic acid by the method of Frakman et al. (1991) which is a 2.4-fold increase over the concentration of the starting material. The invention is clearly effective in producing oleoresins with high carnosic acid contents such as may be preferred by the medical and health industries.

## Claims

1. A process for producing a natural antioxidant oleoresin from a herbal material containing carnosic acid or from a herbal extract containing carnosic acid, the oleoresin having an enhanced concentration of carnosic acid as compared to that of the herbal material or herbal extract, said process comprising the steps of:
a. mixing the herbal material or herbal extract with a 40%-95% solution by weight of ethanol or methanol in water for a sufficient period to dissolve at least 50% by weight of the carnosic acid contained in the herbal material or herbal extract, said sufficient period being established by a quantitative determination;
b. separating and recovering the solution from the insoluble material;
c. adjusting the alcohol concentration of the recovered solution to less than 40%-60% by weight of alcohol in water to cause precipitation of the carnosic acid containing oleoresin; and
d. separating and recovering the precipitated oleoresin containing the carnosic acid from the solution.

2. A process as claimed in claim 1, wherein the herbal material is ground to a fineness of 75% being less than 0.42 mm (40 mesh).

3. A process as claimed in claim 1, wherein the herbal material or herbal extract is mixed with 40%-60% by weight of ethyl alcohol in water for a sufficient period to dissolve at least 50% by weight of the available carnosic acid, followed by adjusting the concentration of the recovered solution to less than 40% by weight of ethyl alcohol by the addition of water.

4. A process as claimed in claim 3, wherein the concentration of the recovered solution is adjusted to less than 20% by weight of ethyl alcohol by the addition of water.

5. A process as claimed in claim 1, wherein the herbal material or herbal extract is mixed with 60%-80% by weight of methyl alcohol in water for a sufficient period to dissolve at least 50% by weight of the available carnosic acid, followed by adjusting the concentration of the recovered solution to less than 60% by weight of methyl alcohol by the addition of water.

6. A process as claimed in claim 5, wherein the concentration of the recovered solution is adjusted to less than 40% by weight of methyl alcohol by the addition of water.

7. A process as claimed in claim 1, wherein the alcohol is food-grade ethyl alcohol.

8. A process as claimed in claim 1, wherein the herbal material is derived from a member of the Labiatae family of plants.

9. A process as claimed in claim 8, wherein the herbal material is the leaves of any of Rosmarinus officinalis, or Salvia officinalis, or Salvia triloba, or Salvia lavandulaefolia.

10. A process as claimed in claim 8, wherein the herbal material is the leaf residue of Rosmarinus officinalis, or Salvia officinalis, or Salvia triloba, or Salvia lavandulaefolia after extraction by steam distillation or subcritical or supercritical carbon dioxide.

11. A process as claimed in claim 8, wherein the herbal material is the oleoresin and natural extract derived from the leaves of Rosmarinus officinalis, or Salvia officinalis, or Salvia triloba, or Salvia lavandulaefolia by extraction with an organic solvent, subcritical or supercritical carbon dioxide.

12. A process as claimed in claim 1, wherein the alcohol concentration of the recovered solution is adjusted to less than 20% by weight of alcohol.

13. A process as claimed in claim 1, wherein the alcohol concentration of the recovered solution is adjusted by distilling off alcohol.

14. A process as claimed in claim 1, further comprising the step of drying the precipitated oleoresin having an enhanced content of carnosic acid to less than a 10% moisture content.

15. An antioxidant composition for use in foods, medicines and cosmetics comprising not less than 40% carnosic acid by weight on a dry matter basis and being substantially devoid of green coloration as judged by qualitative visual examination.

16. An antioxidant composition as claimed in claim 15, dispersed or dissolved in a food, cosmetic or pharmaceutical approved solvent or carrier.

## Patentansprüche

1. Verfahren zur Herstellung eines natürlichen, antioxidierend wirkenden Ölharzes aus einem Carnosinsäure enthaltenden Pflanzenmaterial oder aus einem Carnosinsäure enthaltenden Pflanzenextrakt, wobei das Ölharz eine im Vergleich mit der des Pflanzenmaterials oder des Pflanzenextrakts erhöhte Konzentration an Carnosinsäure besitzt, wobei das Verfahren die folgenden Schritte umfaßt:
a. Mischen des Pflanzenmaterials oder des Pflanzenextrakts mit einer Lösung von 40 - 95 Gew.-% an Ethanol oder Methanol in Wasser während eines Zeitraums, der ausreicht, um mindestens 50 Gew.-% der im Pflanzenmaterial oder Pflanzenextrakt enthaltenen Carnosinsäure aufzulösen, wobei der ausreichende Zeitraum auf einer quantitativen Bestimmung begründet ist;
b. Abtrennen und Rückgewinnen der Lösung vom unlöslichen Material,.
c. Einstellen der Alkoholkonzentration der rückgewonnenen Lösung auf weniger als 40 - 60 Gew.-% an Alkohol im Wasser, um eine Ausfällung des Carnosinsäure enthaltenden Ölharzes zu bewirken; und
d. Abtrennen und Rückgewinnen des die Carnosinsäure enthaltenden, ausgefallenen Ölharzes von der Lösung.

2. Verfahren nach Anspruch 1, worin das Pflanzenmaterial auf eine Feinheit mit 75% unterhalb von 0,42 mm (40 mesh) gemahlen wird.

3. Verfahren nach Anspruch 1, worin das Pflanzenmaterial oder der Pflanzenextrakt mit 40 - 60 Gew.-% an Ethylalkohol in Wasser gemischt wird, während eines Zeitraums, der ausreicht, um mindestens 50 Gew.-% der verfügbaren Carnosinsäure aufzulösen, gefolgt vom Einstellen der Konzentration der rückgewonnenen Lösung auf weniger als 40 Gew.-% an Ethylalkohol, durch die Zugabe von Wasser.

4. Verfahren nach Anspruch 3, worin die Konzentration der rückgewonnenen Lösung durch die Zugabe von Wasser auf weniger als 20 Gew.-% an Ethylalkohol eingestellt wird.

5. Verfahren nach Anspruch 1, worin das Pflanzenmaterial oder der Pflanzenextrakt mit 60 - 80 Gew.-% an Methylalkohol in Wasser gemischt wird, während eines Zeitraums, der ausreicht, um mindestens 50 Gew.-% der verfügbaren Carnosinsäure aufzulösen, gefolgt vom Einstellen der Konzentration der rückgewonnenen Lösung auf weniger als 60 Gew.-% an Methylalkohol, durch die Zugabe von Wasser.

6. Verfahren nach Anspruch 5, worin die Konzentration der rückgewonnenen Lösung durch die Zugabe von Wasser auf weniger als 40 Gew.-% an Methylalkohol eingestellt wird.

7. Verfahren nach Anspruch 1, worin der Alkohol nahrungsmittelgeeigneter Ethylalkohol ist.

8. Verfahren nach Anspruch 1, worin das Pflanzenmaterial von einem Mitglied der Labiatae Pflanzenfamilie herstammt.

9. Verfahren nach Anspruch 8, worin das Pflanzenmaterial die Blätter irgendeines Rosmarinus officinalis oder Salvia officinalis oder Salvia triloba oder Salvia lavandulaefolia sind.

10. Verfahren nach Anspruch 8, worin das Pflanzenmaterial der Blattrückstand von Rosmarinus officinalis oder Salvia officinalis oder Salvia triloba oder Salvia lavandulaefolia nach Extraktion mittels Dampfdestillation oder unterkritischem oder überkritischem Kohlendioxid ist.

11. Verfahren nach Anspruch 8, worin das Pflanzenmaterial das Ölharz und der natürliche Extrakt ist, hergeleitet aus den Blättern von Rosmarinus officinalis oder Salvia officinalis oder Salvia triloba oder Salvia lavandulaefolia durch Extraktion mit einem organischen Lösungsmittel, unterkritischem oder überkritischem Kohlendioxid.

12. Verfahren nach Anspruch 1, worin die Alkoholkonzentration der rückgewonnenen Lösung auf weniger als 20 Gew.-% an Alkohol eingestellt ist.

13. Verfahren nach Anspruch 1, worin die Alkoholkonzentration der rückgewonnenen Lösung durch Abdestillieren von Alkohol eingestellt wird.

14. Verfahren nach Anspruch 1, welches ferner den Schritt des Trocknens des ausgefallenen Ölharzes mit einem gesteigerten Gehalt an Carnosinsäure auf einen Feuchtigkeitsgehalt von weniger als 10% umfaßt.

15. Antioxidierend wirkende Zusammensetzung für die Verwendung in Lebensmitteln, Medikamenten und Kosmetikas, welche nicht weniger als 40 Gew.-%, bezogen auf die Trockenmasse, an Carnosinsäure umfaßt und im wesentlichen frei von einer grünen Färbung ist, was durch eine qualitative visuelle Untersuchung beurteilt wird.

16. Antioxidierend wirkende Zusammensetzung nach Anspruch 15, welche in einem für Lebensmittel, Kosmetika oder pharmazeutische Produkte zugelassenen Lösungsmittel oder Träger dispergiert oder aufgelöst ist.

## Revendications

1. Procédé de préparation d'une oléorésine antioxydante naturelle à partir de plantes ou d'extraits de plantes contenant de l'acide carnosique, ladite oléorésine présentant une plus forte concentration d'acide carnosique que la matière végétale ou l'extrait végétal de base, ledit procédé comprenant les séquences suivantes:
(a) mélanger la matière végétale ou l'extrait végétal avec une solution de 40 à 95% en poids d'éthanol ou de méthanol dans l'eau pendant une durée suffisante pour dissoudre au moins 50% en poids de l'acide carnosique contenu dans la matière végétale ou l'extrait végétal, ladite durée suffisante étant établie par une détermination qualitative,
(b) séparer et récupérer la solution de la matière insoluble,
(c) ajuster la concentration en alcool jusqu'à moins de 40 à 60% en poids de l'alcool dans l'eau afin de provoquer la précipitation de l'oléorésine contenant de l'acide carnosique,
(d) séparer et récupérer l'oléorésine contenant de l'acide carnosique précipitée de la solution.

2. Procédé selon la revendication 1 dans lequel la matière végétale est broyée jusqu'à ce que 75% de la matière présentent un degré de finesse inférieure à 0,42 mm (40 mesh).

3. Procédé selon la revendication 1 dans lequel la matière végétale ou l'extrait végétal est mélangé avec 40 à 60% en poids d'alcool éthylique dans l'eau pendant une durée suffisante pour dissoudre au moins 50% en poids de l'acide carnosique disponible, la concentration de la solution obtenue étant ensuite ajustée par adjonction d'eau jusqu'à présenter moins de 40% en poids d'alcool éthylique.

4. Procédé selon la revendication 3 dans lequel la concentration de la solution obtenue est ajustée par adjonction d'eau jusqu'à présenter moins de 20% en poids d'alcool éthylique.

5. Procédé selon la revendication 1 dans lequel la matière végétale ou l'extrait végétal est mélangé avec 60 à 80% en poids d'alcool méthylique dans l'eau pendant une durée suffisante pour dissoudre au moins 50% en poids de l'acide carnosique, la concentration de la solution obtenue étant ensuite ajustée par adjonction d'eau jusqu'à présenter moins de 60% en poids d'alcool méthylique.

6. Procédé selon la revendication 5 dans lequel la concentration de la solution obtenue est ajustée par adjonction d'eau jusqu'à présenter moins de 40% en poids d'alcool méthylique.

7. Procédé selon la revendication 1 dans lequel l'alcool utilisé est un alcool éthylique de qualité alimentaire.

8. Procédé selon la revendication 1 dans lequel la matière végétale appartient à la famille de plantes des labiées.

9. Procédé selon la revendication 1 dans lequel la matière végétale utilisée est constituée de feuilles d'une sorte quelconque de rosmarinus officinalis, salvia officinalis, salvia triloba ou salvia lavandulaefolia.

10. Procédé selon la revendication 8 dans lequel la matière végétale utilisée est constituée de résidus de feuilles de rosmarinus officinalis, salvia officinalis, salvia triloba ou salvia lavandulaefolia provenant de l'extraction par distillation à la vapeur ou au dioxyde de carbone sous-critique ou supercritique.

11. Procédé selon la revendication 8 dans lequel la matière végétale utilisée est constituée de l'extrait oléorésineux naturel provenant de feuilles de rosmarinus officinalis, salvia officinalis, salvia triloba ou salvia lavandulaefolia après extraction avec un solvant organique ou du dioxyde de carbone souscritique ou supercritique.

12. Procédé selon la revendication 1 dans lequel la concentration en alcool de la solution obtenue est ajustée jusqu'à moins de 20% en poids d'alcool.

13. Procédé selon la revendication 1 dans lequel la concentration en alcool de la solution obtenue est ajustée par distillation de l'alcool.

14. Procédé selon la revendication 1 comprenant en outre une séquence de séchage de l'oléorésine précipitée présentant une teneur en acide carnosique de moins de 10% de la teneur en humidité.

15. Composition antioxydante à usage alimentaire, pharmaceutique ou cosmétique ne contenant pas moins de 40% en poids d'acide carnosique sur la base de la matière sèche et qui est substantiellement dépourvue de couleur verte selon l'appréciation pouvant être faite lors d'un examen qualitatif visuel.

16. Composition antioxydante selon la revendication 15 dispersée ou dissoute dans un solvant ou porteur autorisé pour des produits alimentaires, pharmaceutiques ou cosmétiques.
